# EUROPEAN PATENT APPLICATION

(11) **EP 2 489 280 A2**
(43) Date of publication of application: **22.08.2012**
(21) Application number: 12164144.3
(22) Date of filing: 27.03.2008
(51) Int. Cl.: A23L 1/29, A23L 1/30, A61K 35/74

(54) **Synbiotic to improve gut microbiota**

(30) Priority: 28.03.2007 EP 07105074
(62) Divisional of application: 08718285.3
(71) Applicant: Nestec S.A., 1800 Vevey (CH)
(72) Inventor: Huber-Haag, Karl-Josef, 1009 Pully (CH); Fichot, Marie-Claire, 1807 Blonay (CH); Rochat, Florence, 1820 Montreux (CH); Sprenger, Norbert, 1073 Savigny (CH)
(74) Representative: Corticchiato, Olivier

(57) **Abstract**

The use of a probiotic strain of *Lactobacillus rhamnosus* and an oligosaccharide mixture which comprises 5-70 wt% of at least one N-acetylated oligosaccharide selected from the group comprising GalNAcα1,3Galβ1,4Glc and Galβ1,6GalNAcα1,3Galβ1,4Glc, 20-90 wt% of at least one neutral oligosaccharide selected from the group comprising Galβ1,6Gal, Galβ1,6Galβ1,4Glc Galβ1,6Galβ1,6Glc, Galβ1,3Galβ1,3Glc, Galβ1,3Galβ1,4Glc, Galβ1,6Galβ1,6Galβ1, 4Glc, Galβ1,6Galβ1,3Galβ1,4Glc Galβ1,3Galβ1,6Galβ1,4Glc and Galβ1,3Galβ1,3Galβ1,4Glc and 5-50 wt% of at least one sialylated oligosaccharide selected from the group comprising NeuAcα2,3Galβ1,4Glc and NeuAcα2,6Galβ1,4Glc in the manufacture of a medicament or therapeutic nutritional composition for promoting the development of an early bifidogenic intestinal microbiota in infants delivered by caesarean section is disclosed.

## Description

### Field of the Invention

This invention relates to the administration to infants delivered by Caesarean section of a specific synbiotic mixture, i.e. a probiotic and an oligosaccharide, capable of promoting an early bifidogenic gut microbiota.

### Background to the Invention

Immediately before birth, the gastro-intestinal tract of a baby is thought to be sterile. During the normal process of birth, it encounters bacteria from the digestive tract, skin and environment of the mother and starts to become colonised. The faecal microbiota of a healthy, vaginally-delivered, breast-fed infant of age 2 to 4 weeks which may be taken as the optimum microbiota for this age group is dominated by Bifidobacteria species with some Lactobacillus species and lesser amounts of Bacteroides such as *Bacteriodes fragilis* species, to the exclusion of potential pathogens such as Clostridia. After the completion of weaning at about 2 years of age, a pattern of gut microbiota that resembles the adult pattern becomes established.

It should be noted that, in the healthy, vaginally-delivered, breast-fed infant, Bifidobacteria form the basis of the microbiota accounting for 60-90 % of total bacteria in the infant gut. Breast feeding also promotes intestinal barrier development which, together with bifidobacterial domination leads to enhanced absorption and therefore utilisation of ingested nutrition.

Grönlund *et al* have studied the faecal microbiota of healthy infants born by caesarean section and compared it with that of a comparable group of infants born by vaginal delivery. They concluded that the gut flora of infants born by caesarean delivery may be disturbed for up to six months after the birth. Specifically they noted that the rates of colonisation by Bifidobacteria and Lactobacilli in the caesarean group reached the rates of colonisation in the vaginally delivered group only after one month and ten days respectively (Grönlund et al, "Fecal Microflora in Heathy Infants Born by Different Methods of Delivery: Permanent Changes in Intestinal Flora After Cesarean Delivery", Journal of Pediatric Gastroenterology and Nutrition, 28:19 - 25).

Other workers have suggested that this delayed/aberrant colonisation may have specific consequences in terms of the subsequent development of the infant and have linked these consequences to the differences in gut flora. For example, Laubereau *et al* found that infants born by caesarean section had a greater risk of diarrhoea than vaginally delivered infants (Laubereau et al, Caesarean Section and gastrointestinal symptoms, atopic dermatitis and sensitisation during the first year of life", Arch Dis Child 2004;89:993-997). Negele *et al* found that caesarean delivery may be an additional risk factor for wheezing and allergic sensitisation to food allergens up to the age of two years (Negele et al "Mode of delivery and development of atopic disease during the first 2 years of life" Pediatr Allergy Immunol 2004:15:48 - 54). It has also been suggested that systemic low-grade inflammation and a sub-optimal gut microbiota may also be implicated in the development of obesity (Fantuzzi G. "Adipose tissue, adipokines, and inflammation" J Allergy Clin Immunol. 2005;115:911-919,. Bäckhed F, Ding H, Wang T, et al. "The gut microbiota as an environmental factor that regulates fat storage" Proc Natl Acad Sci USA. 2004;101:15718-15723).

Mother's milk is recommended for all infants. However, in some cases breast feeding is inadequate or unsuccessful for medical reasons or the mother chooses not to breast feed. Infant formulae have been developed for these situations.

In the recent past, certain strains of bacteria have attracted considerable attention because they have been found to exhibit valuable properties for man if ingested. In particular, specific strains of the genera Lactobacilli and Bifidobacteria have been found to be able to colonise the intestine, to reduce the capability of pathogenic bacteria to adhere to the intestinal epithelium, to have immunomodulatory effects and to assist in the maintenance of well-being. Such bacteria are sometimes called probiotics and it has already been proposed to add suitable probiotic bacteria to infant formulae.

Extensive studies have been carried out to identify new probiotic strains. For example, EP 0 199 535, EP 0 768 375, WO 97/00078, EP 0 577 903 and WO 00/53200 disclose specific strains of Lactobacilli and Bifidobacteria and their beneficial effects.

More recently, some concerns have been expressed about the addition of probiotic bacteria to infant formula which is intended as the sole source of nutrition for infants in the first six months of life. These concerns were summarized in the medical position paper from the ESPGHAN Committee on Nutrition entitled "Probiotic Bacteria in Dietetic Products for Infants" (Journal of Paediatric Gastroenterology and Nutrition, 38:365-374).

Another approach to promote the numbers and/or activities of beneficial bacteria in the colon is the addition of prebiotics to foodstuffs. A prebiotic is a non-digestible food ingredient that beneficially affects the host by selectively stimulating the growth and/or activity of one or a limited number of bacteria in the colon, and thus improves host health. Such ingredients are non-digestible in the sense that they are not broken down and absorbed in the stomach or small intestine and thus pass intact to the colon where they are selectively fermented by the beneficial bacteria. Examples of prebiotics include certain oligosaccharides, such as fructooligosaccharides (FOS) and galactooligosaccharides (GOS).

Human milk is known to contain a larger amount of indigestible oligosaccharides than most other animal milks. In fact, indigestible oligosaccharides represent the third largest solid component (after lactose and lipids) in breast milk, occurring at a concentration of 12-15 g/l in colostrum and 5-8 g/l in mature milk. Human milk oligosaccharides are very resistant to enzymatic hydrolysis, indicating that these oligosaccharides may display essential functions not directly related to their calorific value.

As the composition of human milk becomes better understood, it has also been proposed to add prebiotics to infant formula. Various infant formulas supplemented with prebiotics such as mixtures of fructooligosaccharides and galactooligosaccharides for example are commercially available. However, such mixtures approximate only roughly the mixture of oligosaccharides in human milk. Over 100 different oligosaccharide components have been detected in human milk some of which have not been so far detected in animal milks such as bovine milk at all or have been detected only in small quantities. Examples of classes of human milk oligosaccharide that are present in bovine milk and colostrum only in very small quantities or not at all are sialylated and fucosylated oligosaccharides.

Infant formulas containing both probiotics and prebiotics have also been proposed in the continual quest to produce infant formulas which replicate as closely as possible the composition and efficacy of human milk. For example, in WO 2005/000748 it is proposed to supplement infant formula with a mixture of a *Bifidobacterium breve* strain, galactooligosaccharides and fructooligosaccharides (inulin). It is claimed that this mixture, which is described as a synbiotic, regulates the Bifidobacterium population in the colon of infants which consume the supplemented formula to a more "infant-like" population, that is, lower in *Bifidobacterium catenulatum, Bifidobacterium pseudocatenulatum* and *Bifidobacterium adolescentis* species and higher in *Bifidobacterium infantis, Bifidobacterium breve* and *Bifidobacterium longum* species. The mixture is also stated to be useful for the prevention or treatment of an immune condition.

The intestinal microbiota plays an important role in the hydrolysis of indigestible oligosaccharides and polysaccharides to absorbable monosaccharides and activation of lipoprotein lipase by direct action on the villous epithelium. Further, it has recently been demonstrated that human milk contains not only oligosaccharides but also Bifidobacteria. At the same time, genomic studies have convincingly shown that Bifidobacteria present in the gut of breast-fed infants, such as *Bifidobacterium longum,* are specially equipped to utilize breast-milk oligosaccharides as nutrients. *Bifidobacterium longum* is also adapted to the conditions in the large intestine where energy harvest from slowly absorbable carbohydrates takes place.

In short, more and more evidence is emerging which suggests that the establishment of an appropriate intestinal microbiota early in life may be a significant in subsequent healthy development. At the same time the proportion of caesarean deliveries continues to increase reaching as much as 70% of all births in some countries. It is therefore clear that there is a need to provide a means to promote the rapid establishment of an appropriate intestinal microbiota in infants where this does not occur naturally. This need is particularly acute given the current practice of routinely administering prophylactic doses of antibiotics to pregnant women who undergo an elective caesarean delivery.

### Summary of the Invention

As noted above, in the healthy, vaginally-delivered, breast-fed infant, Bifidobacteria form the basis of the microbiota accounting for 60-90 % of total bacteria in the infant gut. The species of Bifidobacteria that are predominantly found in such infants are *Bifidobacterium breve, Bifidobacterium infantis,* and *Bifidobacterium longum.* The present inventors have surprisingly found that coadministration of a specific sub-species of Lactobacillus, namely a probiotic strain of *Lactobacillus rhamnosus* and an oligosaccharide mixture which comprises 5-70 wt% of at least one N-acetylated oligosaccharide selected from the group comprising GalNAcα1,3Galβ1,4Glc and Galβ1,6GalNAcα1,3Galβ1,4Glc, 20-90 wt% of at least one neutral oligosaccharide selected from the group comprising Galβ1,6Gal, Galβ1,6Galβ1,4Glc Galβ1,6Galβ1,6Glc, Galβ1,3Galβ1,3Glc, Galβ1,3Galβ1,4Glc, Galβ1,6Galβ1,6Galβ1,4Glc, Galβ1,6Galβ1,3Galβ1,4Glc Galβ1,3Galβ1,6Galβ1,4Glc and Galβ1,3Galβ1,3Galβ1,4Glc and 5-50 wt% of at least one sialylated oligosaccharide selected from the group comprising NeuAcα2,3Galβ1,4Glc and NeuAcα2,6Galβ1,4Glc synergistically promotes the development of an early bifidogenic intestinal microbiota in infants delivered by caesarean section.

Accordingly the present invention provides the use of a probiotic strain of *Lactobacillus rhamnosus* and an oligosaccharide mixture which comprises 5-70 wt% of at least one N-acetylated oligosaccharide selected from the group comprising GalNAcα1,3Galβ1,4Glc and Galβ1,6GalNAcα1,3Galβ1,4Glc, 20-90 wt% of at least one neutral oligosaccharide selected from the group comprising Galβ1,6Gal, Galβ1,6Galβ1,4Glc Galβ1,6Galβ1,6Glc, Galβ1,3Galβ1,3Glc, Galβ1,3Galβ1,4Glc, Galβ1,6Galβ1,6Galβ1,4Glc, Galβ1,6Galβ1,3Galβ1,4Glc Galβ1,3Galβ1,6Galβ1,4Glc and Galβ1,3Galβ1,3Galβ1,4Glc and 5-50 wt% of at least one sialylated oligosaccharide selected from the group comprising NeuAcα2,3Galβ1,4Glc and NeuAcα2,6Galβ1,4Glc in the manufacture of a medicament or therapeutic nutritional composition for promoting the development of an early bifidogenic intestinal microbiota in infants delivered by caesarean section.

The invention further provides the use of a probiotic strain of *Lactobacillus rhamnosus* and an oligosaccharide mixture which comprises 5-70 wt% of at least one N-acetylated oligosaccharide selected from the group comprising GalNAcα1,3Galβ1,4Glc and Galβ1,6GalNAcα1,3Galβ1,4Glc, 20-90 wt% of at least one neutral oligosaccharide selected from the group comprising Galβ1,6Gal, Galβ1,6Galβ1,4Glc Galβ1,6Galβ1,6Glc, Galβ1,3Galβ1,3Glc, Galβ1,3Galβ1,4Glc, Galβ1,6Galβ1,6Galβ1,4Glc, Galβ1,6Galβ1,3Galβ1,4Glc Galβ1,3Galβ1,6Galβ1,4Glc and Galβ1,3Galβ1,3Galβ1,4Glc and 5-50 wt% of at least one sialylated oligosaccharide selected from the group comprising NeuAcα2,3Galβ1,4Glc and NeuAcα2,6Galβ1,4Glc in the manufacture of a medicament or therapeutic nutritional composition for reducing the risk of subsequent development of allergy in infants delivered by caesarean section.

In a further aspect, the invention provides the use of a probiotic strain of *Lactobacillus rhamnosus* and an oligosaccharide mixture which comprises 5-70 wt% of at least one N-acetylated oligosaccharide selected from the group comprising GalNAcα1,3Galβ1,4Glc and Galβ1,6GalNAcα1,3Galβ1,4Glc, 20-90 wt% of at least one neutral oligosaccharide selected from the group comprising Galβ1,6Gal, Galβ1,6Galβ1,4Glc Galβ1,6Galβ1,6Glc, Galβ1,3Galβ1,3Glc, Galβ1,3Galβ1,4Glc, Galβ1,6Galβ1,6Galβ1,4Glc, Galβ1,6Galβ1,3Galβ1,4Glc Galβ1,3Galβ1,6Galβ1,4Glc and Galβ1,3Galβ1,3Galβ1,4Glc and 5-50 wt% of at least one sialylated oligosaccharide selected from the group comprising NeuAcα2,3Galβ1,4Glc and NeuAcα2,6Galβ1,4Glc in the manufacture of a medicament or therapeutic nutritional composition for preventing or treating diarrhoea in infants delivered by caesarean section.

The invention extends to a method of promoting the development of an early bifidogenic intestinal microbiota in infants delivered by caesarean section comprising providing a therapeutic amount of a probiotic strain of *Lactobacillus rhamnosus* and an oligosaccharide mixture which comprises 5-70 wt% of at least one N-acetylated oligosaccharide selected from the group comprising GalNAcα1,3Galβ1,4Glc and Galβ1,6GalNAcα1,3Galβ1,4Glc, 20-90 wt% of at least one neutral oligosaccharide selected from the group comprising Galβ1,6Gal, Galβ1,6Galβ1,4Glc Galβ1,6Galβ1,6Glc, Galβ1,3Galβ1,3Glc, Galβ1,3Galβ1,4Glc, Galβ1,6Galβ1,6Galβ1,4Glc, Galβ1,6Galβ1,3Galβ1,4Glc Galβ1,3Galβ1,6Galβ1,4Glc and Galβ1,3Galβ1,3Galβ1,4Glc and 5-50 wt% of at least one sialylated oligosaccharide selected from the group comprising NeuAcα2,3Galβ1,4Glc and NeuAcα2,6Galβ1,4Glc to an infant born by caesarean section and in need of the same.

The invention further extends to a method of reducing the risk that an infant delivered by caesarean section will subsequently develop allergy comprising providing a therapeutic amount of a probiotic strain of *Lactobacillus rhamnosus* and an oligosaccharide mixture which comprises 5-70 wt% of at least one N-acetylated oligosaccharide selected from the group comprising GalNAcα1,3Galβ1,4Glc and Galβ1,6GalNAcα1,3Galβ1,4Glc, 20-90 wt% of at least one neutral oligosaccharide selected from the group comprising Galβ1,6Gal, Galβ1,6Galβ1,4Glc Galβ1,6Galβ1,6Glc, Galβ1,3Galβ1,3Glc, Galβ1,3Galβ1,4Glc, Galβ1,6Galβ1,6Galβ1,4Glc, Galβ1,6Galβ1,3Galβ1,4Glc Galβ1,3Galβ1,6Galβ1,4Glc and Galβ1,3Galβ1,3Galβ1,4Glc and 5-50 wt% of at least one sialylated oligosaccharide selected from the group comprising NeuAcα2,3Galβ1,4Glc and NeuAcα2,6Galβ1,4Glc to an infant born by caesarean section and in need of the same.

The invention also extends to a method of preventing or treating diarrhoea in an infant delivered by caesarean section comprising providing a therapeutic amount of a probiotic strain of *Lactobacillus rhamnosus* and an oligosaccharide mixture which comprises 5-70 wt% of at least one N-acetylated oligosaccharide selected from the group comprising GalNAcα1,3Galβ1,4Glc and Galβ1,6GalNAcα1,3Galβ1,4Glc, 20-90 wt% of at least one neutral oligosaccharide selected from the group comprising Galβ1,6Gal, Galβ1,6Galβ1,4Glc Galβ1,6Galβ1,6Glc, Galβ1,3Galβ1,3Glc, Galβ1,3Galβ1,4Glc, Galβ1,6Galβ1,6Galβ1,4Glc, Galβ1,6Galβ1,3Galβ1,4Glc Galβ1,3Galβ1,6Galβ1,4Glc and Galβ1,3Galβ1,3Galβ1,4Glc and 5-50 wt% of at least one sialylated oligosaccharide selected from the group comprising NeuAcα2,3Galβ1,4Glc and NeuAcα2,6Galβ1,4Glc to an infant born by caesarean section and in need of the same.

Without wishing to be bound by theory, the present inventors believe that administration of a probiotic strain of *Lactobacillus rhamnosus* and an oligosaccharide mixture which comprises 5-70 wt% of at least one N-acetylated oligosaccharide selected from the group comprising GalNAcα1,3Galβ1,4Glc and Galβ1,6GalNAcα1,3Galβ1,4Glc, 20-90 wt% of at least one neutral oligosaccharide selected from the group comprising Galβ1,6Gal, Galβ1,6Galβ1,4Glc Galβ1,6Galβ1,6Glc, Galβ1,3Galβ1,3Glc, Galβ1,3Galβ1,4Glc, Galβ1,6Galβ1,6Galβ1,4Glc, Galβ1,6Galβ1,3Galβ1,4Glc Galβ1,3Galβ1,6Galβ1,4Glc and Galβ1,3Galβ1,3Galβ1,4Glc and 5-50 wt% of at least one sialylated oligosaccharide selected from the group comprising NeuAcα2,3Galβ1,4Glc and NeuAcα2,6Galβ1,4Glc to an infant born by caesarean section in some way as yet incompletely understood primes the gastrointestinal tract of the infant to favour subsequent colonisation by those species of Bifidobacteria which are commonly found in the tracts of healthy, vaginally delivered infants. It is thought that this beneficial colonisation reduces the risk of episodes of diarrhoea such as have been shown to afflict infants delivered by caesarean section. It is further thought that the beneficial colonisation reduces the risk of subsequent development of allergy as manifested for example by wheezing and/or sensitisation to food allergens.

It should be noted that it is neither the object nor the effect of such treatment to promote colonisation by the probiotic *Lactobacillus rhamnosus* itself but rather to promote colonisation with other species so as to achieve an early bifidogenic intestinal microbiota comparable with that found in healthy, breast-fed, vaginally-delivered infants.

### Brief Description of the Drawings

**Figure 1** shows the *Staphylococcus aureus* and *Clostridium perfringens* counts in faecal and samples at day 14 of treatment in gnotobiotic mice gavaged with a human baby microbiota; and
**Figure 2** shows *Bifidobacterium breve* and *Bifidobacterium longum* counts in faecal samples at day 14 of treatment in gnotobiotic mice gavaged with a human baby microbiota.

### Detailed Description of the Invention

In this specification, the following terms have the following meanings:-
"early bifidogenic intestinal microbiota" means for an infant up to the age of 12 months an intestinal microbiota which is dominated by Bifidobacteria such as *Bifidobacterium breve, Bifidobacterium infantis,* and *Bifidobacterium longum* to the exclusion of appreciable populations of such species as Clostridia and Streptococci and which is generally comparable with that found in a vaginally-delivered, breast fed infant of the same age.
"infant" means a child under the age of 12 months.
"prebiotic" means a non-digestible food ingredient that beneficially affects the host by selectively stimulating the growth and/or activity of one or a limited number of bacteria in the colon and thus improves host health (Gibson and Roberfroid "Dietary Modulation of the Human Colonic Microbiota: Introducing the Concept of Prebiotics" J. Nutr 125:1401 - 1412).
"probiotic" means microbial cell preparations or components of microbial cells with a beneficial effect on the health or well-being of the host. (Salminen S, Ouwehand A. Benno Y. et al "Probiotics: how should they be defined" Trends Food Sci. Technol. 1999:10 107-10).

All references to percentages are percentages by weight unless otherwise stated.

Suitable probiotic *Lactobacillus rhamnosus* strains include *Lactobacillus rhamnosus* ATCC 53103 obtainable *inter alia* from Valio Oy of Finland under the trade mark LGG and *Lactobacillus rhamnosus* CGMCC 1.3724. A suitable daily dose is from 10e5 to 10e11 colony forming units (cfu), more preferably from 10e7 to 10e10 cfu.

The probiotic *Lactobacillus rhamnosus* is co-administered with an oligosaccharide mixture which comprises 5-70 wt% of at least one N-acetylated oligosaccharide selected from the group comprising GalNAcα1,3Galβ1,4Glc and Galβ1,6GalNAcα1,3Galβ1,4Glc, 20-90 wt% of at least one neutral oligosaccharide selected from the group comprising Galβ1,6Gal, Galβ1,6Galβ1,4Glc Galβ1,6Galβ1,6Glc, Galβ1,3Galβ1,3Glc, Galβ1,3Galβ1,4Glc, Galβ1,6Galβ1,6Galβ1,4Glc, Galβ1,6Galβ1,3Galβ1,4Glc Galβ1,3Galβ1,6Galβ1,4Glc and Galβ1,3Galβ1,3Galβ1,4Glc and 5-50 wt% of at least one sialylated oligosaccharide selected from the group comprising NeuAcα2,3Galβ1,4Glc and NeuAcα2,6Galβ1,4Glc. Such an oligosaccharide mixture is described in more detail in WO2007/090894, the contents of which are incorporated herein by reference and is referred to hereinafter as "the oligosaccharide mixture described above". The term co-adminsitration includes both simultaneous administration of the probiotic *Lactobacillus rhamnosus* and oligosaccharide mixture and sequential administration of the *Lactobacillus rhamnosus* and the oligosaccharide mixture.

Preferably the oligosaccharide mixture described above comprises 10-70 wt% of the specified N-acetylated oligosaccharide(s), 20-80 wt% of the specified neutral oligosaccharide(s) and 10-50 wt% of the specified sialylated oligosaccharide(s). More preferably the mixture comprises 15-40 wt% of the N-acetylated oligosaccharide(s), 40-60 wt% of the other neutral oligosaccharide(s) and 15-30 wt% of the sialylated oligosaccharide(s). A particularly preferred mixture is 30 wt% of the N-acetylated oligosaccharide(s), 50 wt% of the neutral oligosaccharide(s) and 20 wt% of the sialylated oligosaccharide(s).

Alternatively, the oligosaccharide mixture described above may conveniently comprise 5-20 wt% of the specified N-acetylated oligosaccharide(s), 60-90 wt% of the specified neutral oligosaccharide(s) and 5-30 wt% of the specified sialylated oligosaccharide(s)

The oligosaccharide mixture described above may be prepared from one or more animal milks. The milk may be obtained from any mammal, in particular from cows, goats, buffalos, horses, elephants, camels or sheep.

Alternatively the oligosaccharide mixture described above may be prepared by purchasing and mixing the individual components. For example, synthesised galacto-oligosaccharides such as Galβ1,6Galβ1,4Glc Galβ1,6Galβ1,6Glc, Galβ1,3Galβ1,4Glc, Galβ1,6Galβ1,6Galβ1,4Glc, Galβ1,6Galβ1,3Galβ1,4Glc and Galβ1,3Galβ1,6Galβ1,4Glc and mixtures thereof are commercially available under the trade marks Vivinal ® and Elix'or ®. Other suppliers of oligosaccharides are Dextra Laboratories, Sigma-Aldrich Chemie GmbH and Kyowa Hakko Kogyo Co., Ltd. Alternatively, specific glycoslytransferases, such as galactosyltransferases may be used to produce neutral oligosaccharides.

The N-acetylated oligosaccharides may be prepared by the action of glucosaminidase and/or galactosaminidase on N-acetyl-glucose and/or N-acetyl galactose. Equally, N-acetyl-galactosyl transferases and/or N-acetyl-glycosyl transferases may be used for this purpose. The N-acetylated oligosaccharides may also be produced by fermentation technology using respective enzymes (recombinant or natural) and/or microbial fermentation. In the latter case the microbes may either express their natural enzymes and substrates or may be engineered to produce respective substrates and enzymes. Single microbial cultures or mixed cultures may be used. N-acetylated oligosaccharide formation can be initiated by acceptor substrates starting from any degree of polymerisation (DP) from DP=1 onwards. Another option is the chemical conversion of ketohexoses (e.g. fructose) either free or bound to an oligosaccharide (e.g. lactulose) into N-acetylhexosamine or an N-acetylhexosamine containing oligosaccharide as described in Wrodnigg, T.M.; Stutz, A.E. (1999) Angew. Chem. Int. Ed. 38:827-828.

The sialylated oligosaccharides 3'sialyl-lactose and 6'sialyl-lactose may be isolated by chromatographic or filtration technology from a natural source such as animal milks. Alternatively, they may also be produced by biotechnology using specific sialyltransferases either by enzyme based fermentation technology (recombinant or natural enzymes) or by microbial fermentation technology. In the latter case microbes may either express their natural enzymes and substrates or may be engineered to produce respective substrates and enzymes. Single microbial cultures or mixed cultures may be used. Sialyl-oligosaccharide formation can be initiated by acceptor substrates starting from any degree of polymerisation (DP) from DP=1 onwards.

Other probiotic bacteria may be administered with the probiotic *Lactobacillus rhamnosus.* Any lactic acid bacteria or Bifidobacteria with established probiotic characteristics may be used. Suitable probiotic lactic acid bacteria include *Lactobacillus reuteri* ATCC 55730 obtainable from Biogaia or *Lactobacillus paracasei* CNCM I-2116.

Suitable probiotic Bifidobacteria strains include *Bifidobacterium lactis* CNCM I-3446 sold *inter alia* by the Christian Hansen company of Denmark under the trade mark Bb12, *Bifidobacterium longum* ATCC BAA-999 sold by Morinaga Milk Industry Co. Ltd. of Japan under the trade mark BB536, the strain of *Bifidobacterium breve* sold by Danisco under the trade mark Bb-03, the strain of *Bifidobacterium breve* sold by Morinaga under the trade mark M-16V and the strain of *Bifidobacterium breve* sold by Institut Rosell (Lallemand) under the trade mark R0070. A mixture of lactic acid bacteria and Bifidobacteria may be used.

The probiotic *Lactobacillus rhamnosus* and the oligosaccharide mixture described above are preferably administered to the infant immediately after delivery and thereafter for at least the first two months of the life of the infant. More preferably, administration continues until the infant reaches six months of age. The probiotic *Lactobacillus rhamnosus* and the oligosaccharide mixture described above may be conveniently administered in an infant formula.

An infant formula for use according to the present invention may contain a protein source in an amount of not more than 2.0 g/100kcal, preferably 1.8 to 2.0 g/100kcal. The type of protein is not believed to be critical to the present invention provided that the minimum requirements for essential amino acid content are met and satisfactory growth is ensured although it is preferred that over 50% by weight of the protein source is whey. Thus, protein sources based on whey, casein and mixtures thereof may be used as well as protein sources based on soy. As far as whey proteins are concerned, the protein source may be based on acid whey or sweet whey or mixtures thereof and may include alpha-lactalbumin and beta-lactoglobulin in whatever proportions are desired.

The proteins may be intact or hydrolysed or a mixture of intact and hydrolysed proteins. It may be desirable to supply partially hydrolysed proteins (degree of hydrolysis between 2 and 20%), for example for infants believed to be at risk of developing cows' milk allergy. If hydrolysed proteins are required, the hydrolysis process may be carried out as desired and as is known in the art. For example, a whey protein hydrolysate may be prepared by enzymatically hydrolysing the whey fraction in one or more steps. If the whey fraction used as the starting material is substantially lactose free, it is found that the protein suffers much less lysine blockage during the hydrolysis process. This enables the extent of lysine blockage to be reduced from about 15% by weight of total lysine to less than about 10% by weight of lysine; for example about 7% by weight of lysine which greatly improves the nutritional quality of the protein source.

The infant formula may contain a carbohydrate source. Any carbohydrate source conventionally found in infant formulae such as lactose, saccharose, maltodextrin, starch and mixtures thereof may be used although the preferred source of carbohydrates is lactose. Preferably the carbohydrate sources contribute between 35 and 65% of the total energy of the formula.

The infant formula may contain a source of lipids. The lipid source may be any lipid or fat which is suitable for use in infant formulas. Preferred fat sources include palm olein, high oleic sunflower oil and high oleic safflower oil. The essential fatty acids linoleic and α-linolenic acid may also be added as may small amounts of oils containing high quantities of preformed arachidonic acid and docosahexaenoic acid such as fish oils or microbial oils. In total, the fat content is preferably such as to contribute between 30 to 55% of the total energy of the formula. The fat source preferably has a ratio of n-6 to n-3 fatty acids of about 5:1 to about 15:1; for example about 8:1 to about 10:1.

The infant formula may also contain all vitamins and minerals understood to be essential in the daily diet and in nutritionally significant amounts. Minimum requirements have been established for certain vitamins and minerals. Examples of minerals, vitamins and other nutrients optionally present in the infant formula include vitamin A, vitamin B1, vitamin B2, vitamin B6, vitamin B 12, vitamin E, vitamin K, vitamin C, vitamin D, folic acid, inositol, niacin, biotin, pantothenic acid, choline, calcium, phosphorous, iodine, iron, magnesium, copper, zinc, manganese, chloride, potassium, sodium, selenium, chromium, molybdenum, taurine, and L-carnitine. Minerals are usually added in salt form. The presence and amounts of specific minerals and other vitamins will vary depending on the intended infant population.

If necessary, the infant formula may contain emulsifiers and stabilisers such as soy lecithin, citric acid esters of mono- and di-glycerides, and the like.

Preferably, the infant formula will contain the oligosaccharide mixture described above in an amount of from 0.2 to 5 grams per litre of reconstituted formula, preferably 1 to 2 g/l.

The infant formula may optionally contain other substances which may have a beneficial effect such as lactoferrin, nucleotides, nucleosides, and the like.

Both the infant formula and the nutritional formula described above may be prepared in any suitable manner. For example, they may be prepared by blending together the protein, the carbohydrate source, and the fat source in appropriate proportions. If used, the emulsifiers may be included at this point. The vitamins and minerals may be added at this point but are usually added later to avoid thermal degradation. Any lipophilic vitamins, emulsifiers and the like may be dissolved into the fat source prior to blending. Water, preferably water which has been subjected to reverse osmosis, may then be mixed in to form a liquid mixture. The temperature of the water is conveniently about 50°C to about 80°C to aid dispersal of the ingredients. Commercially available liquefiers may be used to form the liquid mixture. The liquid mixture is then homogenised; for example in two stages.

The liquid mixture may then be thermally treated to reduce bacterial loads, by rapidly heating the liquid mixture to a temperature in the range of about 80°C to about 150°C for about 5 seconds to about 5 minutes, for example. This may be carried out by steam injection, autoclave or by heat exchanger; for example a plate heat exchanger.

Then, the liquid mixture may be cooled to about 60°C to about 85°C; for example by flash cooling. The liquid mixture may then be again homogenised; for example in two stages at about 10 MPa to about 30 MPa in the first stage and about 2 MPa to about 10 MPa in the second stage. The homogenised mixture may then be further cooled to add any heat sensitive components; such as vitamins and minerals. The pH and solids content of the homogenised mixture are conveniently adjusted at this point.

The homogenised mixture is transferred to a suitable drying apparatus such as a spray drier or freeze drier and converted to powder. The powder should have a moisture content of less than about 5% by weight.

The probiotic *Lactobacillus rhamnosus* may be cultured according to any suitable method and prepared for addition to the nutritional or infant formula by freeze-drying or spray-drying for example. Alternatively, *Lactobacillus rhamnosus* ATCC 53103 can be bought from Valio Oy of Finland under the trade mark LGG already prepared in a suitable form for addition to food products such as nutritional and infant formulas. The probiotic *Lactobacillus rhamnosus* may be added to the formula in an amount between 10e3 and 10e12 cfu/g powder, more preferably between 10e7 and 10e12 cfu/g powder.

The invention will now be further illustrated by reference to the following examples:-

### Example 1

An example of the composition of a suitable infant formula to be used in the present invention is given below

| Nutrient | per 100kcal | per litre |
|---|---|---|
| Energy (kcal) | 100 | 670 |
| Protein (g) | 1.83 | 12.3 |
| Fat (g) | 5.3 | 35.7 |
| Linoleic acid (g) | 0.79 | 5.3 |
| α-Linolenic acid (mg) | 101 | 675 |
| Lactose (g) | 11.2 | 74.7 |
| Minerals (g) | 0.37 | 2.5 |
| Na (mg) | 23 | 150 |
| K (mg) | 89 | 590 |
| Cl (mg) | 64 | 430 |
| Ca (mg) | 62 | 410 |
| P (mg) | 31 | 210 |
| Mg (mg) | 7 | 50 |
| Mn (µg) | 8 | 50 |
| Se (µg) | 2 | 13 |
| Vitamin A (µg RE) | 105 | 700 |
| Vitamin D (µg) | 1.5 | 10 |
| Vitamin E (mg TE) | 0.8 | 5.4 |
| Vitamin K1 (µg) | 8 | 54 |
| Vitamin C (mg) | 10 | 67 |
| Vitamin B 1 (mg) | 0.07 | 0.47 |
| Vitamin B2 (mg) | 0.15 | 1.0 |
| Niacin (mg) | 1 | 6.7 |
| Vitamin B6 (mg) | 0.075 | 0.50 |
| Folic acid (µg) | 9 | 60 |
| Pantothenic acid (mg) | 0.45 | 3 |
| Vitamin B12 (µg) | 0.3 | 2 |
| Biotin (µg) | 2.2 | 15 |
| Choline (mg) | 10 | 67 |
| Fe (mg) | 1.2 | 8 |
| I (µg) | 15 | 100 |
| Cu (mg) | 0.06 | 0.4 |
| Zn (mg) | 0.75 | 5 |
| *L. rhamnosus* ATCC 53103 | 2.10⁷ cfu/g of powder, live bacteria | |

### Example 2

This example compares the effect of *Lactobacillus rhamnosus* CGMCC 1.3724 with an oligosaccharide ingredient including N-acetylated oligosaccharides, neutral oligosaccharides and sialylated oligosaccharides (referred to hereinafter as CMOS-GOS) on the establishment of an early bifidogenic intestinal microbiota in a gnotobiotic mouse model of caesarean delivery with the effect of the probiotic and oligosaccharide mixture alone and with a control. This model is an appropriate animal model of infants born by caesarean delivery and having a sub-optimal intestinal microbiota in terms of population of Bifidobacteria. In addition to the observation of the size of Bifidobacteria population, this model is also suitable to follow the beneficial effect of the Bifidobacteria as a barrier against potentially pathogenic bacteria like *Clostridium perfringens.*

### Materials and Methods

Germfree C3H female and male mice were purchased from Charles River Laboratories France and shipped to the Nestlé Research Centre in transportation isolators. Animals were transferred to breeding isolators after control of germfree status. Female offspring of this breeding population was used for this study. Animals were then randomly assigned to one of 4 study groups: A, control diet and control drink; B, control diet with 3% oligosaccharide mixture and control drink; C, control diet and probiotic *L. rhamnosus* CGMCC 1.3724 drink; D, control diet with 3% oligosaccharide mixture and probiotic *L. rhamnosus* CGMCC 1.3724 drink.

Animals were kept in two different isolators in cages of 5 animals each. Groups A and B were kept in one isolator and groups C and D were kept in another isolator in order to avoid cross-contamination with *L. rhamnosus* later in the study. Germfree status was monitored weekly in freshly collected faeces from one animal per cage. During this period, the animals were fed the diet AIN-93 basal. At the age of 7 to 8 weeks 2 animals per cage were re-controlled for germfree status and each animal received thereafter by gavage a single dose of 200 µl human baby microbiota cocktail (HBF) as described in Table 1. On the same day diet was changed to AIN-mix (for groups A and C) or AIN-CMOS-GOS (for groups B and D), and drinking water was changed to saline drinking water containing 0.5 % (v/v) MRS (for groups A and B) or saline drinking water containing 0.5 % (v/v) MRS and a final concentration of 2 × 10e7 cfu/ml *L*. *rhamnosus.* This day is considered day -1. The next day (considered here day 0) faeces of each animal were collected freshly and subjected immediately thereafter to microbiota analysis by plate counting.

A cow milk oligosaccharide-enriched sample (CMOS) was prepared starting from an industrial deproteinated and demineralised whey permeate (Lactosérum France, France). Briefly, an ultrafiltration cow milk whey permeate was demineralized on an industrial demineralization line equipped with electrodialysis modules and, anion- and cation exchangers (Lactosérum France). The demineralized whey permeate was then subjected to 2 sequential industrial lactose crystallisation cycles and was subsequently spray dried (Lactosérum France). The resulting powdered modified mother liquor was dissolved in water at 30 % (w/v) and clarified by passing through an active charcoal bed followed by filtration on a 0.22 µm filter (Millipore). The resulting filtrate was loaded onto a preparative Bio-Gel P2 (BioRad) column (50 × 850 mm) run with 20 mM ammonium bicarbonate (NH4HCO3) at a flow rate of 2 ml/min. Fractions containing oligosaccharides and eluting prior to lactose were collected, pooled and lyophilized.

The lyophilised oligosaccharides were blended with commercial galactosyl-oligosaccharides (Vivinal GOS, DOMO Friesland Foods) to obtain a final blend containing about 9 wt % N-acetylated oligosaccharides, about 85 wt % neutral oligosaccharides and about 6 wt % sialylated oligosaccharides. This CMOS-GOS ingredient was incorporated in a AIN-93 semi-synthetic rodent diet to give a final oligosaccharide content of 3 wt%. The control AIN-93 diet was supplemented with glucose and lactose to control for the glucose and lactose that is brought into the CMOS-GOS diet by the used raw materials.

*L. rhamnosus* was prepared from the Nestle Culture Collection. Briefly, NCC4007 was reactivated and grown in MRS (Man Rogosa Sharpe) medium to about 4-5 × 10e8 cfu/ml. Thereafter *L. rhamnosus* was concentrated by centrifugation in its spent MRS medium and diluted to 4 × 10e9 cfu/ml with fresh MRS medium. *L. rhamnosus* was then divided in 1 ml aliquots that were frozen at -80°C until used. Each day 1 freshly defrosted 1 ml aliquot of *L. rhamnosus* in MRS or 1 ml MRS (for groups without *L. rhamnosus*) were introduced in the isolators and dissolved in 200 ml saline and divided equally between four drinking bottles. With an average consumption of 5 ml/day and mouse each animal in the groups with probiotic received about 10e8 cfu *L. rhamnosus* per day.

Faecal samples were collected and analysed on day 14 by plate counting. Briefly, for each mouse 1 faecal pellet was homogenized in 0.5 mL Ringer solution (Oxoid, UK) supplemented with 0.05 % (w/v) L-Cysteine (HCl) and different dilution of the bacterial solution were plated on selective and semi-selective media for the enumeration of specific micro-organisms: Bifidobacteria on Eugom Tomato medium, Lactobacillus on MRS medium supplemented with antibiotics (phosphomycine, sulfamethoxazole and trimethoprime), C. perfringens on NN-agar medium, Enterobacteriaceae on Drigalski medium, and Bacteroides on Shaedler Neo Vanco medium. Plates were incubated at 37 °C under aerobic conditions for 24 h for the counting of Enterobacteriaceae, and under anaerobic conditions during 48 h for Bifidobacteria, Lactobacillus, Bacteroides and C. perfringens.

**Table 1 - Microbiota Composition**

| **strain** | **Colony phenotype on plate** | **concentration administred log(cfu/ml)** |
|---|---|---|
| *Bifidobacterium breve* NCC452 (viv4) | white, big | <2 |
| *Bifidobacterium longum* NCC572 (viv5) | grey, small | <2 |
| *Staphylococcus aureus* FSM124 (viv3) | white, big | 7.0 |
| *Staphylococcus epidermidis* FSM 115 (viv2) | grey, small | 7.0 10 |
| *Escherichia coli* FSM325 (viv1) | | 8.08 |
| *Bacteroides distasonis* FSM24 (viv20) | | 5.0 |
| *Clostridium perfringens* FSMC14 (viv19) | | < 5.0 |

### Results

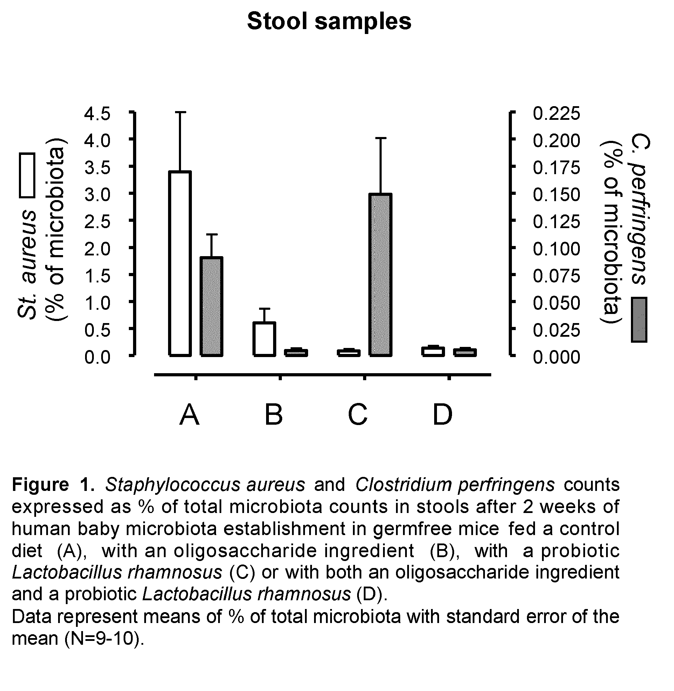

Figure 1 shows the *Staphylococcus aureus* and *Clostridium perfringens* counts in stools two weeks after gavage with HBF for groups A, B, C and D. It may be seen that although counts of *St. aureus* were reduced in both groups B and D and counts of *C. perfringens* were reduced in both groups C and D, it was only in group D that a significant reduction of counts of both pathogens was found.

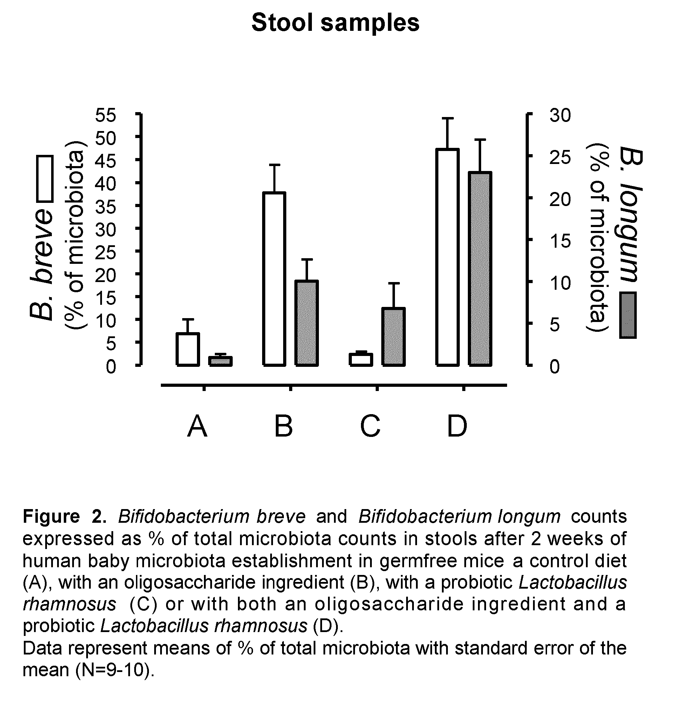

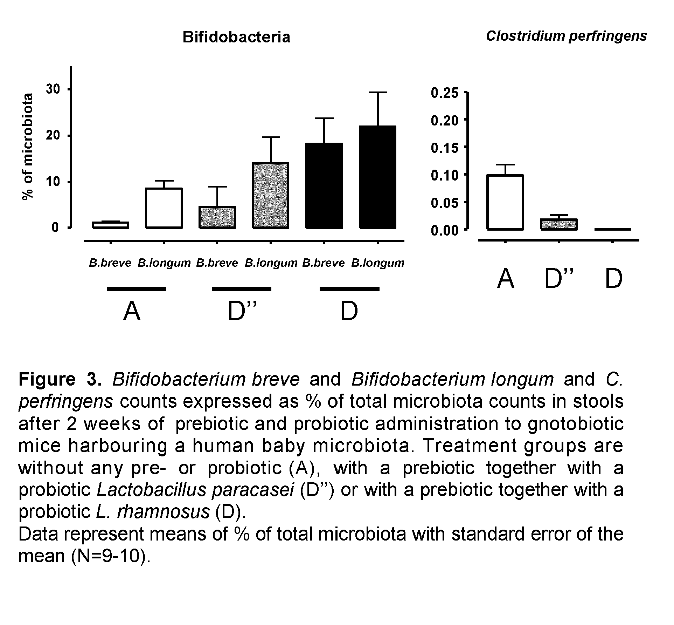

Figure 2 shows the *Bifidobacterium breve* and *Bifidobacterium longum* counts in stools two weeks after gavage with HBF for groups A, B, C and D. It may be seen that both species (which typically dominate the intestinal microbiota of a vaginally-delivered, breast-fed baby) constitute a much larger proportion of the microbiota in group D than in the other groups. In summary, these results show a synergistic effect of the probiotic *Lactobacillus rhamnosus* and the oligosaccharide mixture in promoting colonisation with Bifidobacteria and preventing the establishment of significant populations of *Staphylococcus aureus* and *Clostridium perfringens.*

## Claims

1. The use of a probiotic strain of *Lactobacillus rhamnosus* and an oligosaccharide mixture which comprises 5-70 wt% of at least one N-acetylated oligosaccharide selected from the group comprising GalNAcα1,3Galβ1,4Glc and Galβ1,6GalNAcα1,3Galβ1,4Glc, 20-90 wt% of at least one neutral oligosaccharide selected from the group comprising Galβ1,6Gal, Galβ1,6Galβ1,4Glc Galβ1,6Galβ1,6Glc, Galβ1,3Galβ1,3Glc, Galβ1,3Galβ1,4Glc, Galβ1,6Galβ1,6Galβ1,4Glc, Galβ1,6Galβ1,3Galβ1,4Glc Galβ1,3Galβ1,6Galβ1,4Glc and Galβ1,3Galβ1,3Galβ1,4Glc and 5-50 wt% of at least one sialylated oligosaccharide selected from the group comprising NeuAcα2,3Galβ1,4Glc and NeuAcα2,6Galβ1,4Glc in the manufacture of a medicament or therapeutic nutritional composition for promoting the development of an early bifidogenic intestinal microbiota in infants delivered by caesarean section.

2. The use a probiotic strain of *Lactobacillus rhamnosus* and an oligosaccharide mixture which comprises 5-70 wt% of at least one N-acetylated oligosaccharide selected from the group comprising GalNAcα1,3Galβ1,4Glc and Galβ1,6GalNAcα1,3Galβ1,4Glc, 20-90 wt% of at least one neutral oligosaccharide selected from the group comprising Galβ1,6Gal, Galβ1,6Galβ1,4Glc Galβ1,6Galβ1,6Glc, Galβ1,3Galβ1,3Glc, Galβ1,3Galβ1,4Glc, Galβ1,6Galβ1,6Galβ1,4Glc, Galβ1,6Galβ1,3Galβ1,4Glc Galβ1,3Galβ1,6Galβ1,4Glc and Galβ1,3Galβ1,3Galβ1,4Glc and 5-50 wt% of at least one sialylated oligosaccharide selected from the group comprising NeuAcα2,3Galβ1,4Glc and NeuAcα2,6Galβ1,4Glc in the manufacture of a medicament or therapeutic nutritional composition for reducing the risk of subsequent development of allergy in infants delivered by caesarean section.

3. The use of a probiotic strain of *Lactobacillus rhamnosus* and an oligosaccharide mixture which comprises 5-70 wt% of at least one N-acetylated oligosaccharide selected from the group comprising GalNAcα1,3Galβ1,4Glc and Galβ1,6GalNAcα1,3Galβ1,4Glc, 20-90 wt% of at least one neutral oligosaccharide selected from the group comprising Galβ1,6Gal, Galβ1,6Galβ1,4Glc Galβ1,6Galβ1,6Glc, Galβ1,3Galβ1,3Glc, Galβ1,3Galβ1,4Glc, Galβ1,6Galβ1,6Galβ1,4Glc, Galβ1,6Galβ1,3Galβ1,4Glc Galβ1,3Galβ1,6Galβ1,4Glc and Galβ1,3Galβ1,3Galβ1,4Glc and 5-50 wt% of at least one sialylated oligosaccharide selected from the group comprising NeuAcα2,3Galβ1,4Glc and NeuAcα2,6Galβ1,4Glc in the manufacture of a medicament or therapeutic nutritional composition for preventing or treating diarrhoea in infants delivered by caesarean section

4. The use of any of Claims 1 to 3 wherein the probiotic Lactobacillus rhamnosus is *Lactobacillus rhamnosus* ATCC 53103 or *Lactobacillus rhamnosus* CGMCC 1.3724.

5. The use of any preceding claim, wherein the oligosaccharide mixture comprises 10-70 wt% of the N-acetylated oligosaccharides, 20-80 wt% of the neutral oligosaccharides and 10-50 wt% of the sialylated oligosaccharides.

6. The use of any preceding claim wherein the oligosaccharide mixture comprises 15-40 wt% of the N-acetylated oligosaccharides, 40-60 wt% of the neutral oligosaccharides and 15-30 wt% of the sialylated oligosaccharides.

7. The use of any of Claims 1 to 5, wherein the oligosaccharide mixture comprises 5-20 wt% of the N-acetylated oligosaccharides, 60-90 wt% of the neutral oligosaccharides and 5-30 wt% of the sialylated oligosaccharides.

8. The use of any preceding claim wherein the medicament or therapeutic nutritional composition is administered to the infant immediately after delivery and thereafter for at least 2 months.

9. The use of any preceding claim, wherein the medicament or therapeutic nutritional composition is administered to the infant for at least 6 months after delivery.

10. The use of any preceding claim, wherein the therapeutic nutritional composition is an infant formula.

11. The use of Claim 10 wherein the infant formula comprises between 10e3 and 10e12 cfu of probiotic *Lactobacillus rhamnosus* per gram of composition (dry weight) and 0.2 to 5 grams per litre of reconstituted formula of the oligosaccharide mixture.
